# EUROPEAN PATENT SPECIFICATION

(11) **EP 1 395 171 B1**
(45) Date of publication and mention of the grant of the patent: **26.04.2006**
(21) Application number: 02737868.6
(22) Date of filing: 16.05.2002
(51) Int. Cl.: A61B 5/00

(54) **AN APPARATUS FOR RECORDING BLADDER VOLUME**
VORRICHTUNG ZUR AUFZEICHNUNG DES BLASENVOLUMENS
DISPOSITIF D'ENREGISTREMENT DU VOLUME DE LA VESSIE

(30) Priority: 19.05.2001 DK 200100807; 20.11.2001 DK 200101728
(43) Date of publication of application: 10.03.2004
(73) Proprietor: Kristiansen, Niels Kristian, 8000 Arhus C (DK)
(72) Inventor: Kristiansen, Niels Kristian, 8000 Arhus C (DK)
(74) Representative: Larsen, Hans Ole
(86) International application number: PCT/DK2002/000320
(87) International publication number: WO 2002/094089

(56) References cited:
- US-A- 4 926 871
- US-A- 5 058 591
- US-A- 5 235 985
- US-A- 5 964 710

## Description

The invention relates to an apparatus for recording the bladder volume in humans or animals.

The techniques used till now for determining the volume of the bladder in humans or animals comprise the generally known image diagnostic techniques based on e.g. magnet resonance (MR), X-ray, nuclear medicine or ultrasound.

However, it is characteristic of these techniques that they call for the use of heavy and large equipment which normally requires that the patient is confined to bed during the measurement.

Ultrasound equipment is available which can measure the volume of the bladder in ambulatory patients. This equipment is based on the use of a single ultrasound transducer, which rotates the transducer over the patient's bladder mechanically by means of a gear system driven by an electric motor, such that the entire bladder is scanned, and then, via signal processing in a computer, a three-dimensional (3D) reconstruction of the surface of the bladder and thereby also the volume of the bladder may be created.

The ultrasound measuring equipment driven by an electric motor is portable in the sense that the equipment is fixed in a belt which is tightened around the patient on whom measurements are to be performed. The system, however, is relatively large and heavy because of the mechanical components used for the rotation of the ultrasound transducer and therefore not comfortable for the patients to wear.

Also, the bulky structure with the complicated, mechanical movements means that the apparatus will inevitably generate mechanical noise which may also be unpleasant to the patient.

The mechanically complicated structure will moreover be vulnerable to external impacts, which may occur if the unit is e.g. dropped. The complicated structure will also be expensive to manufacture and require relatively great current maintenance costs.

The operation by an electric motor will require considerable electrical power, which means that batteries for the energy supply must be large and heavy, which in turn makes it difficult and inconvenient to use the apparatus in practice.

Thus, it is a problem of the known techniques that they cannot be used in practice for long term monitoring of ambulatory patients, which is a great clinical need, e.g. in connection with the evaluation of the effect of recently developed medicine.

Both in a research and a clinical connection there is a great need for long term monitoring of the bladder volume in patients where it is attempted to diagnose various bladder and urinary tract disorders.

Another great need for long term monitoring of the volume of the bladder with a user-friendly easily portable apparatus is found in patients who suffer from involuntary noctumal incontinence.

If these individuals could be equipped with a small, simple and non-hampering measuring device which could measure the volume of the bladder continuously and emit a signal when the bladder reached a preselected maximum volume, the individuals would have an alarm that woke them before the urinary incontinence was initiated, so that the individuals could urinate in time and thereby avoid the inconvenience of the otherwise occurring urinary incontinence.

Urinary nocturnal incontinence is a great problem for up to 15% of all children aged 3 -10 years.

USA 5,235,985 discloses an apparatus for recording bladder volume in humans comprising three identical phased-array transducers, the transducers being arranged in a portable fixture.

It is an object of the present invention to enable continuous measurement of the volume of the bladder in humans or animals who or which move about free and easy or are at sleep, and to obtain a measurement that provides more detailed detection possibilities of the bladder.

The object of the invention is achieved by an apparatus of the type defined in claim 1, i.e. an apparatus for recording the bladder volume in humans or animals, said apparatus comprising at least three identical phased-array transducers, each of said transducers having a plurality of transducer elements, said transducers being arranged in a portable fixture and being disposed on a circle having a centre which is positionable at the mass centre of the bladder, and further comprising a calculation unit which is capable of calculating the volume of the bladder on the basis of the signals from the transducer elements.

As stated in claim 2, the apparatus may contain two transducers which are positioned on said circle with a spacing of 90°, whereby the scanning planes for the transducers can cover the entire bladder, allowing subsequent 3D reconstruction of the external shape and volume of the bladder.

As stated in claim 3, the apparatus may also be provided with three or more phased-array ultrasound transducers which are positioned equidistantly on said circle.

As stated in claim 4, the fixture of the apparatus has integrated therein electronic circuits which are used for controlling the transducers and for processing the signals from the transducers, which electronic circuits may be connected to an input medium, such as a keyboard, and an output medium, such as a sound generator or vibrator, thereby allowing the recorded signals to be processed. In addition, the electronic circuits may input and output data, if the input and output media are connected thereto. The electronic circuits may thus be used for emitting e.g. an acoustic alarm signal, if the bladder volume exceeds a preselected maximum.

When, as stated in claim 5, the fixture is a belt which has integrated therein electronic circuits which are capable of wirelessly communicating with external units, such as computers or mobile telephones, the keyboard of the mobile telephone may e.g. be used as an input medium for inputting data, e.g. to describe how large the volume of a bladder must be as a basis for the triggering of an alarm.

In the example involving communication with a mobile telephone, an alarm signal may of course also be given via the mobile telephone in the form of a call or vibrator activation.

Finally, as stated in claim 6, the apparatus is powered from batteries which are integrated in the fixture, and which may be rechargeable.

The invention will now be explained more fully with reference to the drawing, in which
- fig. 1: shows the torso of an individual with an attached apparatus having a total of seven ultrasound transducers for recording the volume of the bladder,
- fig. 2: shows an example of the positions of seven transducers for recording the bladder volume,
- figs. 3.1, 3.2: show the transducer arrangement of fig. 2 in an enlarged section illustrating the structure of the individual phased-array ultrasound transducers in detail,
- fig. 4: shows the scanning planes from two selected transducers from a measuring apparatus that contains seven identical phased-array ultrasound transducers,
- fig.5: shows a cross-section of the body of an individual whose bladder volume is measured using phased-array ultrasound transducers. The figure shows the scanning plane in the body of the individual for one transducer,
- fig. 6: shows a cross-section of the body of an individual whose bladder volume is measured in accordance with the present invention. The figure shows how all the transducers are directed toward the same point in the body of the individual being measured, while
- fig. 7: shows the position of the transducers in the situation where precisely two transducers are used.

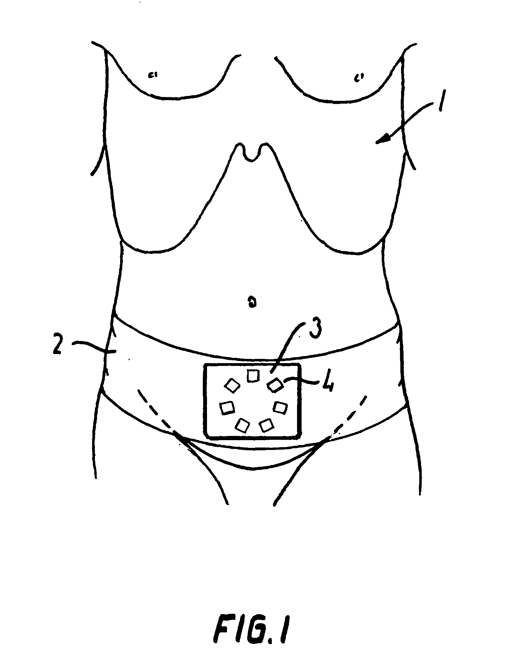

Fig. 1 shows the torso of an individual 1 who is to have his bladder volume recorded. With this end in view, the individual is provided with a fixture in the form of a belt 2, which may also be integrated in the waistband of the pants. The apparatus 3 for the bladder measurement is arranged in the belt, based on a plurality of identical ultrasound transducers 4 of the phased-array type.

It is characteristic of the phased-array ultrasound transducers that these can perform a scanning sweep in a plane without mechanical rotation of the transducer, as the transducer is composed of multiple piezoelectric crystals arranged in parallel which are capable of emitting signals in various angles by time-delayed, individual excitation in a plane.

The fixture, in which the apparatus is mounted, is positioned such that the centre of the transducers is present at the mass centre of the bladder.

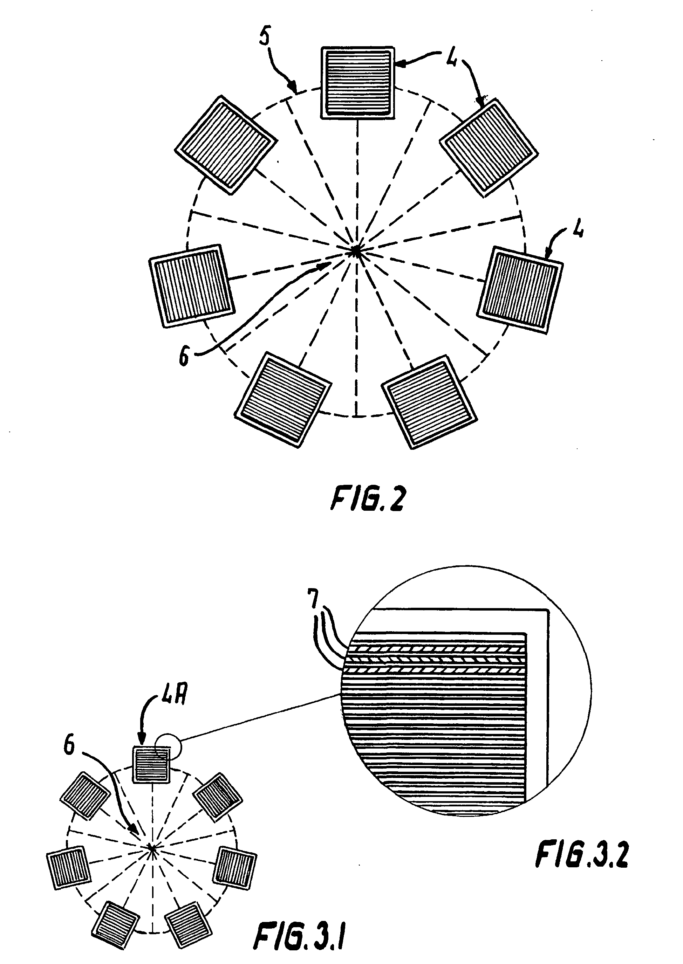

An enlarged section of the transducers of fig. 1 is shown in fig. 2, where, as will be seen, there is a total of seven identical transducers 4 which are disposed equidistantly on the same circle 5 having a centre 6 which is intended to be positioned at the mass centre of the bladder being measured. When three or more transducers are used, it is optimum in terms of measuring that the transducers are positioned equidistantly on the circle 5. This provides the best imaging of the bladder, as the scanning planes from the individual transducers are distributed as best as possible, thereby achieving the best distribution of input data in a polar system of coordinates. Of course, it is without importance whether the data collection takes place in polar or Cartesian coordinates, it being simple to transform between the two forms of coordinates so that these may be used freely where it is most expedient.

It is a prerequisite for the measurement of the bladder volume to operate expediently that the scanning planes of the ultrasound transducers are coordinated. Fig. 3.1 shows the positions of seven transducers in a measurement setup, and a section of a transducer 4A of the identical transducers is shown in an enlarged view in fig. 3.2. The phased-array transducers scan in a plane which extends in the same direction as the one in which the piezoelectric crystals 7 are positioned side by side. In fig. 3.1, the transducer 4A, a section of which is shown in an enlarged view in fig. 3.2, will scan in a plane which extends along the line between the transducer 4A and the common centre 6. Thus, it is a prerequisite for the optimum operation of the measurement setup that all the transducers are oriented such that they scan in a plane which follows the radius from the common centre 6 to the central axis of the individual transducers. As mentioned, the scanning planes follow the drawn tracks 7 and propagate in the other dimension into the body of the individual being measured.

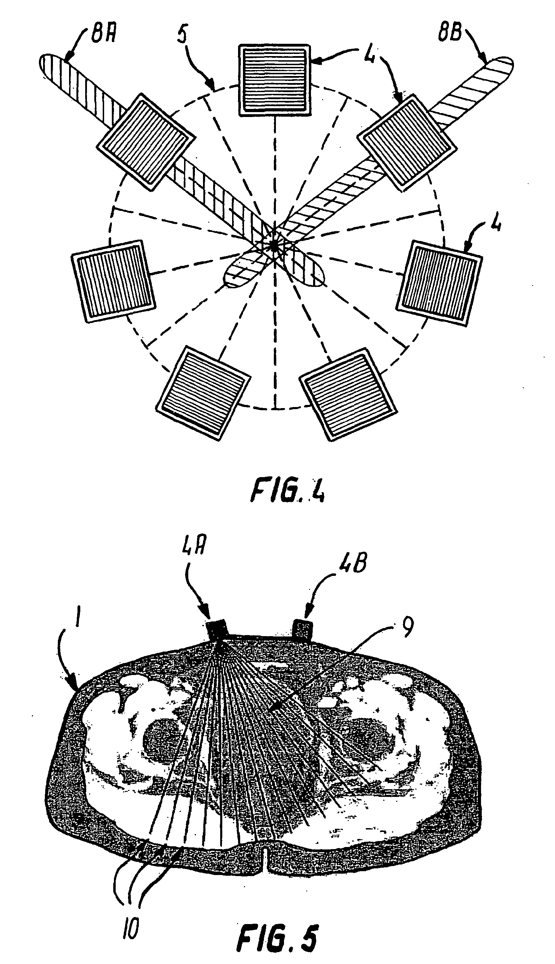

It is shown in fig. 4 how the scanning planes extend from two arbitrarily selected transducers from a measurement system of seven transducers. The scanning planes of the two transducers are shown in 8A and 8B. respectively, and, for each transducer, follow the previously mentioned radius line that extends from the central axis of the individual transducers to the common centre 6.

Fig. 5 shows a section of the body 1 of the individual where the volume of the bladder 9 is to be recorded. Two transducers 4A and 4B and the scanning lines 10 in the body 1 of the individual, shown for the transducer 4A, are drawn in the figure.

Tests have shown that the best result of an ultrasound scanning of the bladder is achieved in the scanning planes where the scanning lines penetrate the surface of the bladder perpendicularly relative to it. After image reconstruction, this gives the most informative image of the bladder with a minimum of shadows that blur otherwise essential image information.

A perpendicular penetration of ultrasound signals relative to the surface of the bladder thus gives a better signal/noise ratio than images which are reconstructed on the basis of ultrasound signals which have not penetrated the bladder perpendicularly relative to the surface of the bladder.

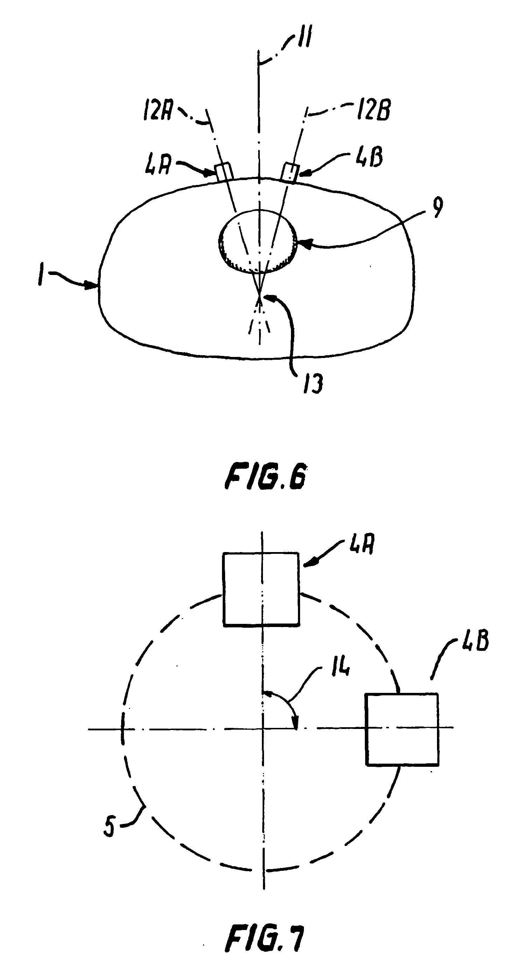

It is part of the present invention to optimize the signal/noise ratio in the signals which are used for calculating the volume of the bladder by expedient orientation of the surface of the individual transducers relative to the bladder to be measured. Fig. 6 shows a schematic section of fig. 5, where two transducers 4A and 4B are arranged on the body 1 for the purpose of imaging the bladder 9. As mentioned before, the transducers are disposed on a circle having a centre through which a central axis 11 is drawn in fig. 6, said central axis having the same spacing from all the transducers at all points. To achieve the optimum position of the transducers, these are oriented such that the central lines 12A and 12B, which extend through the centre of the transducers, will intersect the central axis 11 at the same point in a direction toward the bladder 9 whose volume is to be recorded. The shown orientation of the transducers ensures that each transducer scans the bladder at an intended, right angle relative to the surface of the bladder with the consequent positive influence on the signal/noise ratio.

In the case where just two transducers are used, it is not optimum, however, to position these equidistantly on the circle 5. Fig. 7 shows the optimum position of two transducers 4A and 4B which, as shown, are positioned 90 degrees offset relative to each other, cf. angle 14. When just two transducers are used, the 90 degrees offset position will cause the scanning planes from the two transducers to be perpendicular to each other. This ensures that the scanning planes may be related to their respective axes in a Cartesian system of coordinates corresponding to e.g. the X-axis and the Y-axis, the third axis being common to both scannings from which it is prior art to reconstruct a 3D image.

The apparatus according to the present invention may advantageously comprise electronic circuits for controlling the transducers, including excitation of these as well as data collection from them. Electronic circuits in the form of signal processors or microprocessors will be capable of calculating the volume of the bladder on the basis of algorithms which are simple to develope for a person skilled in the art.

By connecting an input medium, e.g. in the form of a keyboard, the apparatus may be pre-programmed, also to emit a signal when the volume of the bladder exceeds an optional level.

Also, the apparatus may advantageously be equipped with electronic circuits for wireless communication with external units, such as computers or mobile telephones.

When the apparatus is connected wirelessly to external units, data may be exchanged between these, which allows remote-controlled programming of the apparatus and alarm to the external units.

Since the apparatus does not contain energy-intensive mechanical and electro-mechanical components, the apparatus may be powered by small and light-weight batteries,

The described invention enables continuous monitoring of the bladder volume in ambulatory or sleeping individuals.

The measurements are substantially without discomfort to the users because of the low weight and the small volume. Since the apparatus does not contain mechanical gears or electric motors, it is noise-free and sturdy as well as simple and inexpensive to manufacture.

## Claims

1. An apparatus (3) for recording the bladder volume in humans or animals, said apparatus comprising at least three identical phased-array transducers (4), each of said transducers having a plurality of transducer elements, said transducers being arranged in a portable fixture and being disposed on a circle (5) having a centre which is positionable at the mass centre of the bladder, and further comprising a calculation unit which is capable of calculating the volume of the bladder on the basis of the signals from the transducer elements.

2. An apparatus for recording the bladder volume according to claim 1, **characterized in that** two of said transducers (4) are positioned on said circle (5) with a spacing of 90 degrees.

3. An apparatus for recording the bladder volume according to claim 1, **characterized in that** three or more transducers (4) are positioned equidistantly on said circle.

4. An apparatus for recording the bladder volume according to one or more of claims 1 - 3, **characterized in that** the fixture has integrated therein electronic circuits which are used for controlling the transducers (4) as well as processing the signals from the transducers, which electronic circuits may be connected to an input medium, such as a keyboard, and an output medium, such as a sound generator or vibrator.

5. An apparatus for recording the bladder volume according to one or more of claims 1 - 3, **characterized in that** the fixture is a belt (2) which has integrated therein electronic circuits which are capable of wirelessly communicating with external units, such as computers or mobile telephones.

6. An apparatus for recording the bladder volume according to one or more of claims 1 - 5, **characterized in that** the apparatus is powered from batteries which are integrated in the fixture, and which may be rechargeable.

## Revendications

1. Appareil (3) destiné à enregistrer le volume de la vessie d'humains ou d'animaux, ledit appareil comprenant au moins trois sondes à déphasage (4) identiques, chacune desdites sondes présentant une pluralité d'éléments de sonde, lesdites sondes étant agencées dans une fixation portable et étant disposées sur un cercle (5) présentant un centre qui peut être positionné au centre de gravité de la vessie, et comprenant en outre une unité de calcul qui est capable de calculer le volume de la vessie sur la base des signaux provenant des éléments de sonde.

2. Appareil destiné à enregistrer le volume de la vessie selon la revendication 1,
**caractérisé en ce que** deux desdites sondes (4) sont positionnées sur ledit cercle (5) avec un espacement de 90 degrés.

3. Appareil destiné à enregistrer le volume de la vessie selon la revendication 1,
**caractérisé en ce que** trois sondes (4) ou plus sont positionnées à équidistance sur ledit cercle.

4. Appareil destiné à enregistrer le volume de la vessie selon l'une ou plusieurs des revendications 1 à 3, **caractérisé en ce que** la fixation intègre en son sein des circuits électroniques qui sont utilisés pour contrôler les sondes (4) ainsi que traiter les signaux provenant des sondes, lesquels circuits électroniques peuvent être raccordés à un support d'entrée, tel qu'un clavier, et un support de sortie, tel qu'un générateur de son ou un vibreur.

5. Appareil destiné à enregistrer le volume de la vessie selon l'une ou plusieurs des revendications 1 à 3, **caractérisé en ce que** la fixation est une ceinture (2) qui intègre en son sein des circuits électroniques qui sont capables de communiquer sans fil avec des unités externes, telles que des ordinateurs ou des téléphones mobiles.

6. Appareil destiné à enregistrer le volume de la vessie selon l'une ou plusieurs des revendications 1 à 5, **caractérisé en ce que** l'appareil fonctionne grâce à des batteries qui sont intégrées dans la fixation, et qui peuvent être rechargeables.

## Patentansprüche

1. Vorrichtung (3) zum Aufzeichnen des Blasenvolumens von Menschen oder Tieren, wobei die Vorrichtung mindestens drei identische Wandler (4) in phasengesteuerter Anordnung aufweist, wobei jeder der Wandler eine Vielzahl von Wandlerelementen besitzt, wobei die Wandler in einer tragbaren Halterung angeordnet sind und auf einem Kreis (5) angeordnet sind, der eine Mitte besitzt, die an der Massenmitte der Blase positionierbar ist, und weiterhin eine Berechnungseinheit aufweist, die dazu geeignet ist, das Volumen der Blase auf der Basis der Signale von den Wandlerelementen zu berechnen.

2. Vorrichtung zum Aufzeichnen des Blasenvolumens nach Anspruch 1, **dadurch gekennzeichnet, dass** zwei der Wandler (4) auf dem Kreis (5) mit einer Beabstandung von 90 Grad positioniert sind.

3. Vorrichtung zum Aufzeichnen des Blasenvolumens nach Anspruch 1, **dadurch gekennzeichnet, dass** drei oder mehr Wandler (4) äquidistant auf dem Kreis positioniert sind.

4. Vorrichtung zum Aufzeichnen des Blasenvolumens nach einem oder mehreren der Ansprüche 1-3, **dadurch gekennzeichnet, dass** die Halterung darin elektronische Schaltungen integriert besitzt, die dazu verwendet werden, die Wandler (4) zu steuern ebenso wie die Signale von den Wandlern zu verarbeiten, wobei die elektronischen Schaltungen mit einem Eingabemedium, wie beispielsweise einem Tastenfeld, und einem Ausgabemedium, wie beispielsweise einem Klanggenerator oder einem Vibrator, verbunden sein können.

5. Vorrichtung zum Aufzeichnen des Blasenvolumens nach einem oder mehreren der Ansprüche 1 - 3, **dadurch gekennzeichnet, dass** die Halterung ein Band (2) ist, das darin integriert elektronische Schaltungen besitzt, die dazu geeignet sind, drahtlos mit externen Einheiten, wie beispielsweise Computern oder Mobiltelefonen, zu kommunizieren.

6. Vorrichtung zum Aufzeichnen des Blasenvolumens nach einem oder mehreren der Ansprüche 1 - 5, **dadurch gekennzeichnet, dass** die Vorrichtung von Batterien versorgt wird, die in der Halterung integriert sind und die wieder aufladbar sein können.
